(19) European Patent Office — Europäisches Patentamt / European Patent Office / Office européen des brevets

(11) **EP 1 080 683 A2**

(12) **EUROPEAN PATENT APPLICATION**

(43) Date of publication:
07.03.2001 Bulletin 2001/10

(51) Int. Cl.⁷: **A61B 5/00**

(21) Application number: **00118665.9**

(22) Date of filing: **29.08.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **30.08.1999 US 151319 P**
**04.11.1999 US 434142**

(71) Applicant:
**CAS MEDICAL SYSTEMS, INC.**
**Branford, CT 06450 (US)**

(72) Inventor: **Benni, Paul**
**Middletown, CT 06457 (US)**

(74) Representative: **Hirsch, Peter**
**Klunker Schmitt-Nilson Hirsch**
**Winzererstrasse 106**
**80797 München (DE)**

(54) **Laser diode optical transducer assembly for non-invasive spectrophotometric blood oxygenation monitoring**

(57) A non-invasive near infrared spectrophotometric monitoring transducer assembly includes a housing member which is adhered directly on a patient's skin. The housing member contains a cluster of laser diodes which emit infrared light in different wavelengths which are necessary to monitor the level of blood oxygenation in the patient. The assembly also contains a light guide mounted in the housing member, which light guide contacts the patient's skin when the housing member is adhered to the patient's skin. The light guide controls the spacing between the laser diodes and the patient's skin, and therefore controls the intensity of the area on the patient's skin which is illuminated by the laser diodes. The light guide also provides a planar interface between the assembly and the patient's skin which the laser diodes are illuminating. The light guide may include a light attenuator and/or diffuser, such as a filter, which allows the usage of higher energy laser diodes to produce a safer lower energy light field on the patient's skin. The assembly also includes an LED safety device which is operable to turn the laser diodes off in the event that the assembly accidentally becomes detached from the patient's skin. The housing member contains a photodiode assembly which detects the infrared light at a second location on the skin to determine light absorption. The photodiode assembly is preferably shielded from ambient EMI interference. The housing may be associated with a disposable sterile hydrogel coated adhesive envelope, or pad, which when applied to the patient's skin will adhere the housing to the patient's skin. The transducer assembly will thus be reusable, and skin-contacting part of the device, i.,e., the envelope or pad can be discarded after a single use.

FIG. 2

EP 1 080 683 A2

## Description

TECHNICAL FIELD

This invention relates to an improvement in a non-invasive near-infrared spectroscopic (NIRS) optical transducer assembly. More particularly, this invention relates to a NIRS optical transducer assembly which can be reusable, and which can provide an accurately sized and attenuated laser light field on a patient's skin.

BACKGROUND ART

Near-infrared spectroscopy (NIRS) is an optical spectrophotometric method of continually monitoring tissue oxygenation. The NIRS method is based on the principle that light in the near-infrared range (700 to 1,000 nm) can pass easily through skin, bone and other tissues but, within these wavelengths, hemoglobin has specific absorption spectra, dependent upon its oxidation state, i.,e., oxygenated-hemoglobin ($HbO_2$); and deoxygenated-hemoglobin (Hb). By using light sources that transmit near-infrared light at specific different wavelengths, and measuring changes in transmitted or reflected light attenuation, oxygenation concentration changes of $HbO_2$ and Hb can be monitored.

Total hemoglobin is the summation of the two states of hemoglobin ( Total Hb = $HbO_2$ + Hb ), and is proportional to relative blood volume changes, provided that the hematocrit or hemoglobin concentration of the blood is unchanged. The most valuable aspect of NIRS is that it allows one to continually monitor cerebral oxygenation levels in an adult or neonate, especially in diseased conditions, in which oxygenation levels in the brain can be compromised, leading to brain damage or death.

It is known that near-infrared light passes through the skin and the skull of a neonate readily, and is absorbed by certain biological molecules the brain. Near-infrared spectroscopy (NIRS) detects oxygenation changes in biological tissue (brain, muscle, or other organs) mainly at the micro circulation level (capillaries, arterioles, and venuoles) based on different absorption characteristics of the chromophores oxyhemoglobin ($HbO_2$) and deoxyhemoglobin (Hb) in the near-infrared spectrum (700-1,000 nm). Average tissue penetration is 2-3 cm with sub-second time resolution. Relative changes of the concentrations of $HbO_2$ and Hb can be quantified by using the modified Beer-Lambert Law, which takes into account the optical attenuation in a highly scattering medium like biological tissue. The modified Beer-Lambert Law can be expressed as:

$$A = -\log (I/I_o)_L = a_L \times C \times d \times B + G \qquad \text{(Equation 1)}$$

wherein A is the optical attenuation in tissue at wavelength L (units: optical density $\approx$ OD); $I_o$ is the incident light intensity (units: $W/cm_2$); I is the detected light intensity; $a_L$ is the wavelength-dependent absorption coefficient of the chromophore (units: OD x $cm^{-1}$ x $\mu M^{-1}$); C is the concentration of chromophore (units: $\mu M$); d is the light source-to-detector distance (units: cm); B is the light scattering differential path length factor (unit less); and G is a factor relating to tissue geometry and scattering of light (units: OD).

Absolute measurement of chromophore concentration is very difficult because G is unknown. However, over a reasonable measuring period of several hours to days, G remains constant, allowing for the measurement of relative changes of chromophore from a zero reference baseline. Thus, if time $t_2$ is an arbitrary time after the start of the optical measurement at $t_1$ (baseline), differential attenuation $\Delta A$ can be calculated, canceling out the variables G and $I_o$, providing that they remain constant. The objective is to determine changes in chromophore concentration [$\Delta C = C(t_2) - C(t_1)$] from $\Delta A$. derived from the equation:

$$\Delta A = -\log (I_2/I_1)_L = a_L \times \Delta C \times d \times B \qquad \text{(Equation 2)}$$

NIRS algorithms that are designed to calculate the relative changes of more than one chromophore use the multi variate form of Equation 2. To distinguish between, and to compute relative changes in, oxyhemoglobin ($\Delta HbO_2$) and in deoxyhemoglobin ($\Delta Hb$), a minimum of two different wavelengths, preferably from narrow spectral bandwidth light sources, like laser diodes, are preferred. The units of $\Delta HbO_2$ and $\Delta Hb$ are in $\mu$moles per liter of tissue ($\mu M$) which is determined from a dimensional analysis of Equation 1.

U.S. Patents Nos. 5,217,013; 5,465,714; 5,482,034; and 5,584,296 utilize light-emitting diodes (LEDs) as the NIRS light source, and two photodiodes in a flexible adhesive surface mountable housing for their NIRS systems. The problem with using LEDs as the light source in NIRS instruments is that the spectral band width of LEDs is much larger (20 - 50 nm) than that of laser diodes (<1 nm). Narrow spectral band width light sources are much easier to utilize in non-invasive NIRS algorithm development thereby enabling the derivation of more reliable and accurate computations of biological parameters. Thus, using multiple narrow spectral bandwidth light sources that are of low power, incorporated directly in the NIRS probe housing, is an improvement over broad bandwidth LED light sources in NIRS probes.

Rigid annular light guides such as those described in U.S. Patents Nos. 5,465,714 and 5,584,296 have an open-air hollow interior that does not have light attenuating capabilities. The walls of these annular light guides reflect light from, or to, the optical component. This configuration is not desirable in a NIRS application because light intensity on the skin is not predetermined, since skin deforms when pressed by the open air hollow annular housing, with no mechanism to predict or maintain constant skin deformation over the optical measuring period.

The mounting systems of the prior art NIRS probe housings are one component systems, in which the probe housing surface interfacing the subject's skin is a single use adhesive, so that these probes are designed for one time use and are discarded after use. This approach is costly and wasteful.

U. S. Patents Nos. 4,321,930; 4,380,240; 4,510,938; and 5,353,791 describe optical transducers that are coupled to laser light sources by means of fiber optics. U. S. Patent No. 5,353,791 also describes a transparent spacer which acts as a laser light guide. The transparent spacer is preferably provided with elasticity means. No specific light intensity attenuation structure is described in these patents to purposely reduce the level of the laser light beam cast upon the patient's skin.

It would be desirable to have a reusable NIRS transducer assembly having onboard laser diodes and the ability to accurately control the energy level and size of a laser light field cast upon a patient's skin during use of the assembly.

DISCLOSURE OF THE INVENTION

This invention relates to an improved transducer assembly for use in near-infrared spectroscopy (NIRS) of human patients. Two or more laser diodes of different wavelengths emittance, and one or more photodiodes are contained in a flexible housing which can be easily and securely attached to a patient's head, or some other part of the body. A rigid light guide is disposed between the laser diodes and the patient's skin for the purpose of decreasing the intensity of the laser light field cast on the skin by the laser diodes, so that laser safety requirements set forth by the American National Standards Institute for laser exposure to human tissues are satisfied. The rigid light guide may also include light attenuation capabilities so as to allow for a wider selection of available laser diodes in the 5 to 40 mW power range to be utilized. Thus, laser diodes of desired wavelengths and package size, but not available with similar power outputs, can be designed with the appropriate light guide in a NIRS probe optical transducer. The light attenuation properties of the rigid light guide allow the design of a Class 1 laser product which incorporates Class 3 laser diodes in the 5 to 40 mW power range. Class 1 laser product classification is important because regulatory and safety requirements are much less stringent for Class 1 devices. The rigid light guide also satisfies the required constant light intensity output of the spectrophotometric measuring system during a measuring period, by maintaining a constant laser diode emitter-to-skin distance.

The NIRS transducer assembly of this invention may consist of two separable components, the NIRS probe housing containing the laser diodes and photodiode(s) described above; and a disposable adhesive envelope or pad which is used to mount the NIRS transducer assembly housing easily and securely to the patient's skin, It is economically more feasible to use a non-disposable NIRS transducer housing with a disposable envelope rather than a disposable NIRS transducer housing, while maintaining all of the advantages of single use, disposable transducer applications, especially in a health care environment, in which sanitation and sterilization requirements are paramount.

As with prior art laser diode-coupled fiber optic NIRS headband transducer systems, the advantage of using a light radiation with a narrow spectral bandwidth (< 1-3 nm) is maintained. The utilization of lower power laser diodes in the transducer assembly of this invention reduces the manufacturing cost of the NIRS transducer assembly, as well as eliminating the cost of the fiber optic components of the prior art. The prior art fiber optic components are also fragile and prone to breakage.

The lower power laser diodes in the transducer assembly of this invention may be pulsed at a low duty cycle so that self heating will not occur, thereby minimizing temperature-induced wavelength shifting, as well as maintaining the laser diode metal casing cool to the touch. The higher power laser diode/fiber optic NIRS transducer systems of the prior art require temperature control systems and heat sinking to dissipate self-generated heat. With negligible self-heating, the low power laser diodes used in the transducer assembly of this invention can be placed in close proximity with the patient's skin by means of an optically transmissive and electrically insulative thin rigid light guide material, such as glass, or a clear plastic material. A thermistor may be placed next to the laser diodes to compensate for any power and wavelength shifting by providing temperature information to the NIRS system processor.

The laser diode light guide, which is placed over the output window of the laser diode, has several functions. One function is to decrease the intensity of laser light on the skin of a subject undergoing spectrophotometric monitoring by taking advantage of the elliptical conical radiation characteristics of the laser diode. Thus, with an increasing separation distance (r) between the laser diode emitter chip and the skin surface, the intensity (power/area) of the laser light decreases by a factor of $r^2$. This is important for designing a laser diode-based optical transducer assembly meant to be directly applied to a human forehead, or some other part of the human body. To assure safety for the skin and tissue, the laser diode optical transducer assembly must be designed to operate within the limitations which are imposed by the "Maximum Permissible Exposure (MPE)" values set forth by the American National Standard for the safe use of lasers (ANZ136.1-1993). As will be noted hereinafter, the distance r can be manipulated by the use of appropriate prisms placed in the transducer housing.

One or more photodiodes are also incorporated in the NIRS transducer assembly housing, separated from the laser diodes by from about 10 to more than about 60 mm, depending on the size of the subject being monitored. For a typical adult human head, it is believed that at least about 45 to about 50 mm separation distance is desirable for adequate brain blood oxygenation monitoring, using a reflection mode type of the NIRS transducer assembly. Multiple photodiodes can be used to monitor different depths blood oxygenation in the patient, or can be used as reference detectors for algorithms that compensate for the scalp component of the detected signals. For neonates, shorter separation distances between the laser diodes and the photodiodes of around 20 mm can be used for reflection mode monitoring, or large distances over 60 mm can be used for trans-cranial mode NIRS transducer assembly. Photodiodes with larger surface areas can be used as the laser diode-to-photodiode separation distances increase, to compensate for the decreasing light levels detected from larger separation distances or lower power light sources.

A photodiode preamplifier, placed next to the photodiode, or farther away as a separate assembly, allows for amplification of the detected low light level signal, and then provides the amplified signal to the NIRS system processor.

A safety light emitting diode (LED) placed in close proximity with the laser diodes serves as a part of a laser-safety interlock, which further minimizes the possibility of laser light exposure to personnel using the NIRS system. The purpose of the laser-safety-interlock is to inhibit laser diode pulsing in case the NIRS probe is not securely attached to the subject. The LED (wavelength 600-980 nm.) will operate prior to the initiation of laser diode pulsing. LED light levels are monitored by the photodiode to detect secure NIRS probe attachment. Ambient light monitoring circuitry will also assist in the determination. The laser diodes will not pulse until the laser-safety-interlock indicates secure probe attachment during the beginning of the monitoring session. Accidental probe detachment will automatically shut down the laser diodes.

Any event that indicates probe detachment will require user intervention to reattach the probe and to reset the laser-safety-interlock before laser diode pulsing can resume.

A partially optically transparent, and electrically conductive shield which surrounds the photodiode can be used to attenuate ambient electromagnetic interference (EMI) noise which is otherwise transmitted to the photodiode. A window in the shield exposes the photodiode's photosensitive surface to detected light from the laser diodes. The optically transparent electrically conductive shield may include a thin metal wire screen, an electrically conductive transparent coating, or the like.

The use of a disposable adhesive envelope or pad for the purpose of securing the NIRS transducer assembly housing to the subject's skin, the transducer assembly housing itself containing the laser diodes and photodiode(s), renders the transducer assembly housing reusable from patient to patient. The disposable adhesive envelope or pad can be pre-sterilized thereby providing additional protection to the subject. The disposable envelope or pad will also protect the NIRS transducer assembly housing from any residue from the subject, allowing the NIRS probe housing surface to remain uncontaminated, thus making it safer and easier to reuse.

Different NIRS transducer assemblies are designed to be interchangeable with different NIRS system processors/monitors by incorporation of custom laser diode drivers and encoded calibration parameters in a connector housing. The connector housing interfaces the electro-optical components of the transducer housing by shielded, multiple lead cables. The NIRS system processor has an interface port for the connector housing. The connector housing may contain customized laser diode automatic power control (APC) drivers, which are individually adjusted to provide a predetermined laser diode output power. By providing encoded calibration parameters in the connector housing, the NIRS system processor can determine the characteristics of each individual NIRS by a decoding mechanism, calibrating the NIRS algorithm to provide accurate computation with different transducer assemblies. A customized photodiode preamplifier may also be included in the connector housing, providing flexibility in photodiode selection.

Enabling but non-essential details of the invention will become more readily apparent from the following detailed description of the invention when taken in conjunction with the accompanying drawings in which:

BRIEF DESCRIPTION OF THE DRAWINGS

FIG. 1 is a plan view of one embodiment of a reflective NIRS transducer assembly which is formed in accordance with this invention;

FIG. 2 is a side sectional view of the transducer assembly of FIG. 1;

FIG.3 is an enlarged sectional view of the LED-laser diode portion of the transducer assembly of FIG. 2;

FIG. 4 is an enlarged sectional view showing the relationship between the transducer assembly laser diode and light guide and the patient's skin;

FIG. 4A is a sectional view similar to FIG. 4, but showing a modification of the laser diode portion which includes light deflecting prisms;

FIG.5 is an enlarged sectional view of the photodiode portion of the transducer assembly of FIG. 2 showing details of the EMI shielding mechanisms for the photodiodes;

FIG.6 is an enlarged plan view of the EMI shielded photodiode portion of the transducer assembly of FIG. 2;

FIG.7 is a plan view of a disposable self-adhesive envelope which is designed for use in containing the transducer assembly housing of FIG. 1;

FIG.8 is a plan view of a disposable self-adhesive pad which is designed for use in connection with the transducer assembly housing of FIG. 1;

FIG. 9 is a plan view of another embodiment of a transmissive NIRS transducer assembly which is formed in accordance with this invention;

FIG. 10 is a plan view similar to FIG. 1 but showing an alternative embodiment of the NIRS assembly of this invention;

FIG. 11 is a sectional view similar to FIG. 2 but showing the embodiment of FIG. 10;

FIG. 11A is a view of the light source portion of the embodiment of FIG. 11;

FIG. 12 is a schematic view of a NIRS transducer assembly formed in accordance with this invention which is associated with a connector housing and a NIRS system processor;

FIG. 13 is schematic view of a NIRS transducer assembly which is similar to FIG. 12, but showing partitioned laser power control drivers and the pre-amp portion of the system located between the connector housing and the assembly housing; and

FIG. 14 is a schematic view of a NIRS transducer assembly which is similar to FIG. 13, but which incorporates features of the embodiments of FIGS. 10 and 11.

DETAILED DESCRIPTION THE INVENTION

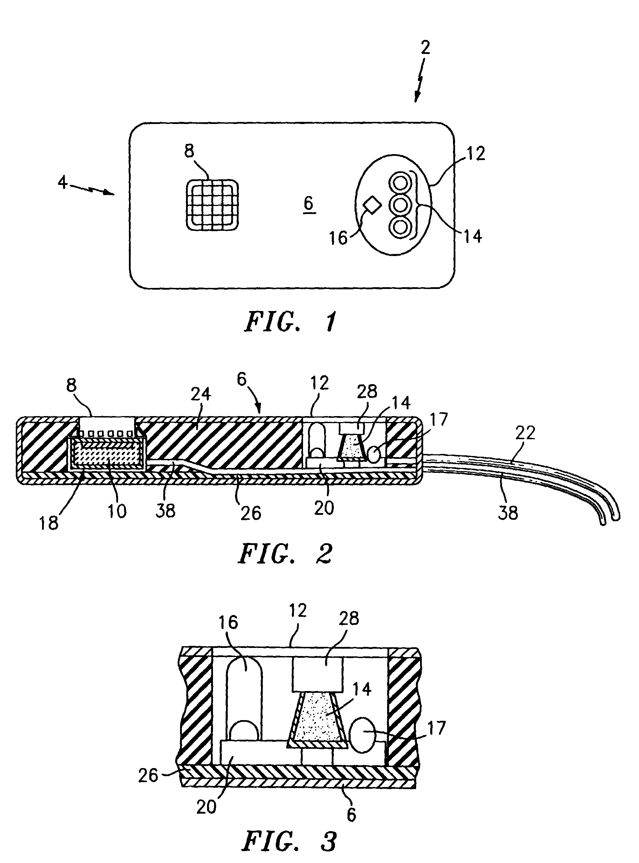

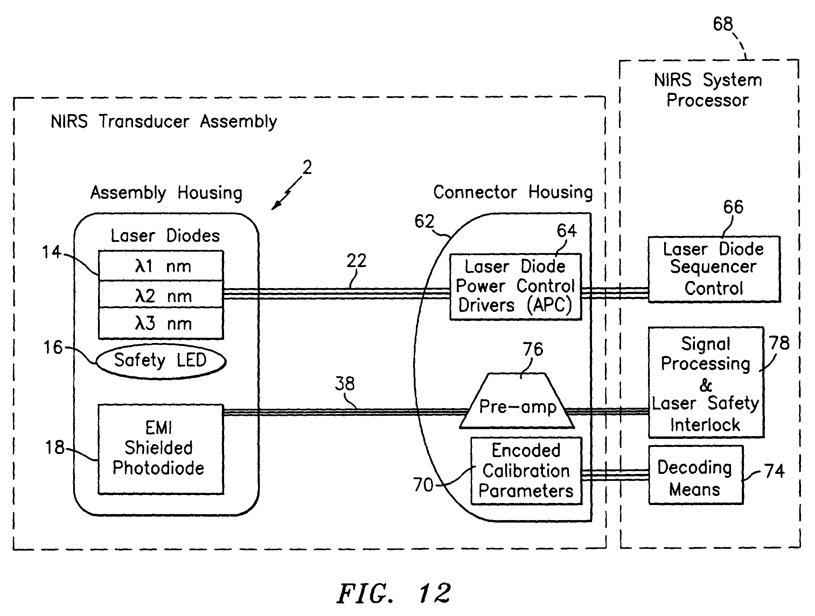

Referring now to the drawings, there is shown in FIGS. 1 and 2 an embodiment of a reflective-type NIRS transducer assembly which is denoted generally by the numeral 2. The transducer assembly 2 includes a housing 4 which contains the electronic components of the transducer assembly 2. The housing 4 includes a flexible light-shielding wall 6 having a first window 8 which overlies an EMI shielded photodiode assembly 18 which is disposed in the housing 4. The light-shielding wall 6 also includes a second window 12 which overlies the skin-illuminating laser diode cluster 14 and a safety LED 16. The laser diode cluster 14, the safety LED 16, and and a thermistor 17 are mounted on a PC board 20 which interfaces with a multi-lead shielded cables 22. The thermistor 17 monitors laser diode temperature to allow the NIRS algorithm to compensate for possible power and wavelength shifting. The housing 4 also includes a flexible, electrically and optically insulative body 24 can be formed from rubber or some other suitable elastomer, and a flexible support structure 26 on which the laser diode cluster PC board 20, and the EMI-shielded photodiode assembly 18 are mounted. Another shielded cable 38 interconnects the photodiode assembly 18 and the NIRS system processor assembly via an interface connector housing (See FIG. 12). The NIRS system processor analyzes the results of the NIRS reflectance data gathered by the photodiode 10.

Referring now to FIG. 3, details of the safety LED 16 and the laser diode cluster 14 mountings are shown. The laser diode cluster 14 is connected to a rigid light guide 28 which provides surface-to-surface contact between the laser diode cluster 14 and the patient's skin S. The light guide 28 is rigid so that when it is pressed against the patient's skin during the monitoring of blood oxygen, the surface S of the skin is flattened, and the distance between the laser diode cluster 14 and the skin surface S is constant across the entire illuminated area of the skin. The LED 16 emits an infrared light beam in the wavelength range of about 600 to about 980 nm. The intensity of the reflected LED IR light is detected by the photodiode 10, along with the reflected light from the laser diode cluster 14. The intensity of the detected LED light is used to monitor the status of the interface of the transducer assembly 2 and the patient's skin S. In the event that the intensity of the detected LED light declines, the system controller will assume that the transducer assembly 2 has come detached from the patient's skin S. That assumption having been made, the system controller will turn the laser diode cluster 14 off, and sound an alarm so that an attendant can verify the status of the transducer assembly 2.

The laser intensity utilized will be far below the threshold indicated in ANSIZ136.1-1993 for cases of accidental eye exposure. This is because of the conical radiation pattern, low duty cycle, and the low power level of the laser light. The incorporation of a laser safety interlock further minimizes the possibility of laser light exposure to personnel using the NIRS system. The safety interlock system inhibits laser diode pulsing immediately if the NIRS assembly is not securely attached to the subject. The laser diode safety circuit (LSC) consists of the light emitting diode 16 (LED, L 600-980 nm.) located close to the laser diodes 14 (or other laser light source) on the NIRS assembly. LED light levels sensed by the photodiode 10 is used to detect secure NIRS assembly attachment, especially In low ambient light conditions. The LED 16 is included in the multiplexed laser diode pulsing scheme, and light detected is processed in the same way as the laser light, except that the LED 16 is active at all times when a NIRS assembly is attached to the system monitor. A window comparator processor determines if the LED light detected is in a predetermined range to indicate secure assembly attachment. Low LED detected light levels would indicate that the laser light potentially is radiating in free space, or is obstructed. High LED detected light levels would indicate that the assembly is loose, by assuming that the light is reflecting off the skin or an object to the photodiode 10, without passing through biological tissue.

For normal, daytime ambient light conditions, a further laser interlock is incorporated by monitoring the DC level of the detected light. This is obtained by low pass filtering the photodiode preamplifier output. When the ambient light detected reaches a predetermined level, indicating possible NIRS assembly detachment, laser operation is inhibited, The laser diodes 14 will not pulse until the LSC indicates secure assembly attachment during the beginning of the monitoring session. Accidental assembly detachment will automatically shut down the laser diodes 14. Any event that indicates assembly detachment will require user intervention to reattach the assembly 2 and to reset the LSC before laser diode pulsing can resume.

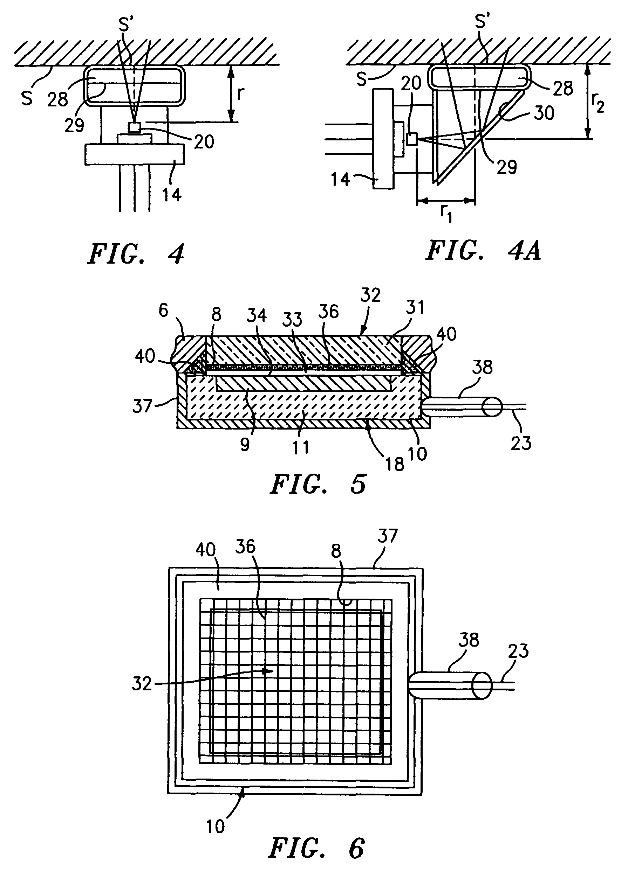

Referring to FIGS. 4 and 4A, there are shown two different light guide arrangements whereby the light guide 28 can control the distance "r" between the laser diode emitter 20 and the surface S of the patient's skin. First of all, it is noted that the planar surface S' of the light guide 28, when pressed against the patient's skin S, will flatten the contacted area of the patient's skin S so that the distance "r" between the laser diode emitter 20 and the patient's skin S is constant for the entire skin area which is illuminated by the light guide 26. In the embodiment shown in FIG. 4, the light from a laser diode emitter 20 passes directly through the light guide 28, so that the distance "r" is simply the straight line distance from the laser diode emitter 20 to the surface S' of the light guide 28. In the embodiment shown in FIG. 4A, the laser diode 14 casts its light field onto a light deflecting prism surface 30, which in turn directs the light field through the light guide 28 and onto the patient's skin S. The inclusion of the prism 30 increases the effective length of "r" since "$r = r_1 + r_2$". Thus, with the prism arrangement, one can effectively reduce the light intensity of the laser diode 14 field which is delivered to the patient's skin S. The result of including the prism 30 is an increase in the length of the light path and an enlargement of the area of the beam that is delivered to the patient's skin S. Thus it may be possible to utilize higher energy laser diodes when the system includes the light deflecting prisms. In order to attenuate the intensity of the laser light field imposed on the skin S, the light guide 28 may include a selective filter 29 which is shown in both FIGS. 4 and 4A. The selective fitter 29 may consist of a light-attenuating neutral density filtering element, or a light-diffusive element, such as a milky white semi-transparent plastic material, or both, in combination.

Laser diodes usually contain a small Internal monitoring photodiode 21 for automatic power control (APC) feedback control. Normally, a small amount of laser light is directed internally in the laser diode from the emitter 20 to the monitoring photodiode 21 by reflection or direct transmission. When using laser diodes in a NIRS probe, laser light may also be reflected back into the laser diode from the skin's surface, which causes a change in power output. This is because the current generated by the monitoring photodiode is usually very small and the additional skin-back-reflected light can increase the photodiode current substantially. As a result, the laser power output may fluctuate unpredictably due to variable amounts of back-reflected light. The rigid light guide 28 maintains laser output stability by attenuating the variable back-reflected laser light from the skin surface by the use of a selective filter 29. Also, the slightly reflective properties of the light guide 28 reflect a predetermined additional amount of light back to the monitoring photodiode 21, which further minimizes the effect of currents generated from variable back-reflections by increasing the monitoring photodiode to a higher, less corruptible level.

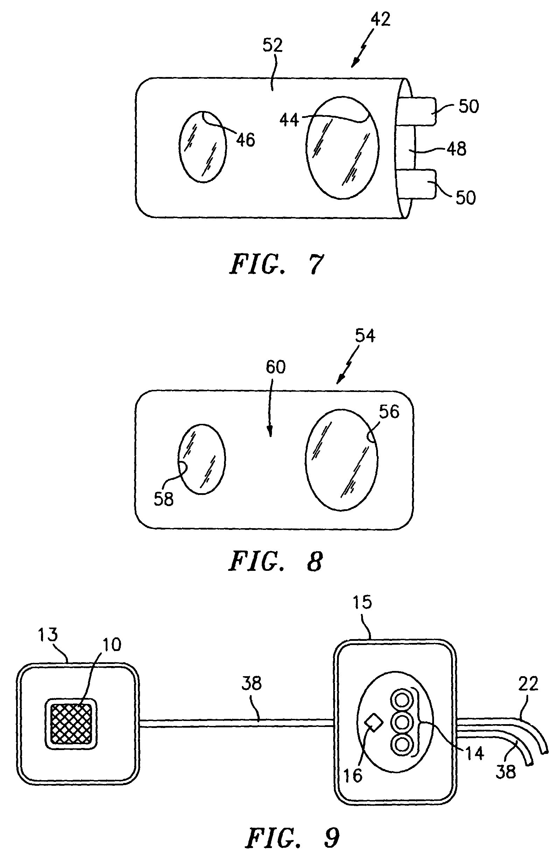

FIGS. 5 and 6 illustrate an ambient EMI shielding arrangement for the photodiode detector window 8. An EMI shielded and optically transparent pane 32 of predetermined thickness, i.,e., at least about 1 mm, is positioned over the light sensitive surface 34 of the photodiode detector 10. The photodiode detector 10 includes a light-sensitive element 9 which is disposed in a ceramic cup 11. A wire mesh 36 is embedded between two optically transparent members 31 and 33 which form the pane 32. The mesh 36 will allow at least about 60% of the reflected light from the laser diodes 14 to reach the photodiode 10. A non-porous EMI shield 37, such as an electro-conductive metal foil, surrounds the non-light sensitive parts of the housing, including the photodiode lead 23. An electrically conductive gasket 40, such as silicone paste, adhesive, or other similar material, is used to create an electrical interface between the wire mesh 36 of the EMI shielded pane 32 and the non-porous EMI shield 37. This type of shielding reduces undesirable EMI generated noise, and improves the signal to noise ratio of the photodiode by two different methods. The wire mesh 36 and non-porous electro-conductive material 37 combination creates a Faraday Cage around the photodiode 10, while allowing light to reach the photodiode 10 light-sensitive surface 34. By using an optically transparent pane 32 of predetermined thickness, i.,e., greater than about 1 mm, further EMI attenuation is attained by increasing the photodiode-to-biological tissue separation distance. This reduces the electromagnetic coupling and generated noise currents between the photodiode light sensitive surface 34 and the biological tissue, such as human skin. To construct the EMI shielded optical sensor, commercially available EMI shielded windows from Chomerics (Woburn, MA) can be used. Chomerics "Emi Clare"™ GP 70 EMI shielded windows provide 60 to 70 % light transmission, with different pane thicknesses of: 1.66; 2.00; and 3.00 mm being available. The transparent panes 32 and the mesh 36 need to be sized to close the window 8 and cover the light-sensitive surface 34 of the photodiode 10, which surface 34 can range in size from four square mm to one hundred square mm.

In an alternative EMI shielding embodiment, the wire mesh 36 could be placed directly over the photodiode light sensitive surface 34, and an optically transparent, electrically insulating pane 32 having a thickness of greater than about 1 mm, formed from a material such as glass, could then be placed over the wire mesh 36.

Referring now to FIGS. 7 and 8, there are shown two different types of discardable or disposable adjunct adherence devices that can be used in conjunction with the transducer assembly 2. FIG. 7 shows an envelope 42 which can be used to house the assembly 2. The envelope 42 has two transparent plastic windows 44 and 46 which are sized and positioned to be in registry with the laser diode assembly 14 and the safety LED 16, and with the photodiode assembly 10, respectively. The transducer assembly 2 is inserted into the envelope 42 through an opening 48. The envelope 42 also includes several clasps 50 that are used to secure the assembly 2 in place in the envelope 42. The surface 52 of the envelope 42 is formed from, or coated with, an adhesive material. The envelope 42 is formed from a light-impermeable material such as rubber or black plastic. This prevents light from directly traveling between the laser diodes and the photodiode (i.e., no optical shunting) which could occur with a transparent envelope.

FIG. 8 shows a disposable or discardable adhesive pad 54 which can be releasably adhered to the transducer assembly 2 and to the patient's skin. The pad 54 is also formed from rubber or black plastic, and includes opposed adherent surfaces such as 60 which enable the pad 54 to be adhered to the transducer assembly 2 and to the patient's skin S. The pad 54 has two transparent plastic windows 56 and 58 which are sized and positioned to be in registry with the laser diode assembly 14 and the safety LED 16, and with the EMI shielded photodiode assembly 18, respectively. The envelope 42 and the pad 54 are both sterile prior to use, and can be discarded after being removed from the patient.

FIG. 9 shows a transmissive embodiment of a NIRS transducer assembly formed in accordance with this invention which is suitable for use with neonates. In this embodiment, the EMI shielded photodiode assembly 18 is contained on one shielded pod 13, and the laser diode assembly 14 and LED 16 are contained in another pod 15. The pods 13 and 15 are connected by the cable 38, and the cable 22 extends from the pod 15 to the controller. The length of the cable 38 is sufficient to allow the pods 13 and 15 to be attached to opposite sides of the neonate's head to allow the transmissive monitoring of cerebral blood oxygenation.

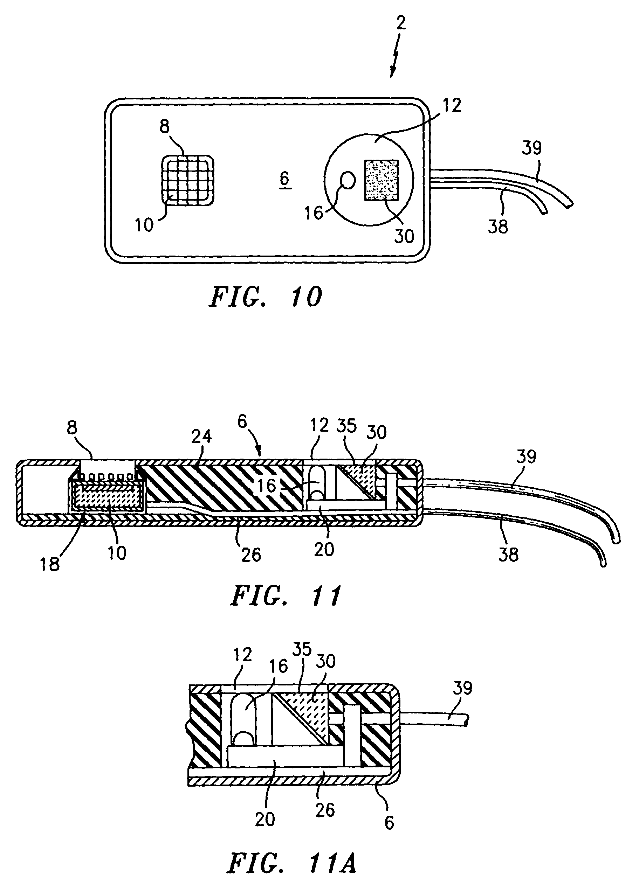

FIGS. 10, 11 and 11A illustrate an alternative embodiment of the NIRS assembly 2 which employs three remotely located laser diodes and fiber optics 39 which direct light from the remote laser diodes to a prism 30 located adjacent to the LED 16. The photodiode window 8 is shielded in the same manner as described above. In another alternative embodiment, an additional fiber optic may be used to direct light from a remote safety LED to the prism 30. The prism 30 will be preferably provided with a light diffusing surface 35 which minimizes back reflection of the light beams from the fiber optics.

FIG. 12 illustrates the components of a NIRS transducer assembly connector housing 62 which enables interchangeability of the laser diode power outputs. The housing 62 contains laser diode automatic power control drivers 64 which interface with the laser diodes 14 by means of the cable 22. A laser diode sequencer control 66 forms a portion of the NIRS system processor 68 and provides multiplexed pulsing of the laser diodes 14. Each laser diode power output is adjusted by its respective automatic power control driver. There are three different laser diode pin-out configurations available, each of which requires different types of automatic power control drivers. Thus, incorporation of the automatic power control drivers 64 in the connector housing 62 provides flexibility in the selection of the laser diodes 14.

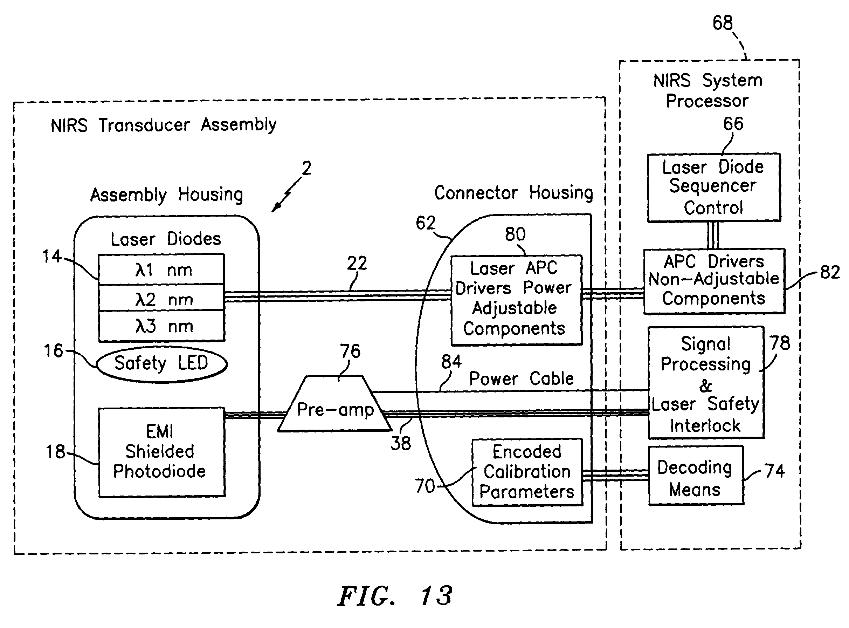

In an alternative connector assembly embodiment shown in FIG. 13, the automatic power control drivers 64 may be separated into adjustable components 80, and non-adjustable components 82. In this embodiment, the adjustable components 80 are disposed in the connector housing 62 and the non-adjustable components 82 are incorporated into the NIRS system processor 68. The adjustable components 80 include variable potentiometers which are used in adjusting the laser power output. The non-adjustable components 82 consist of fixed semiconductor and discrete electronic components, and are typically resistors and capacitors. FIG. 13 also shows an alternative placement of the photo diode preamplifier 76 interfaced to the cable 38 between the connector housing 62 and the assembly housing 2. A preamplifier power cable 84 supplies power to the preamplifier 76.

The connector housing 62 also contains a calibration parameter encoding mechanism 70 which provides the NIRS system processor with necessary information relating the the NIRS transducer assembly characteristics. The calibration parameters include: laser diode wavelength and power; laser diode to photodiode separation distance; and other information as needed about the characteristics of the individual NIRS transducer assembly being employed. The calibration parameters may be encoded by the use of resistors of predetermined values, programmable read only memory devices, bar codes, or other suitable encoding instrumentalities. The encoded information is transmitted to a decoder 74 in the system processor 68.

A photodiode preamplifier 76 may be located in the connector housing 62 for providing amplification of the light level signal from the EMI-shielded laser diode assembly 14 and transmitting the amplified signal to a signal processing and laser safety interlock control portion 78 of the NIRS system processor 68.

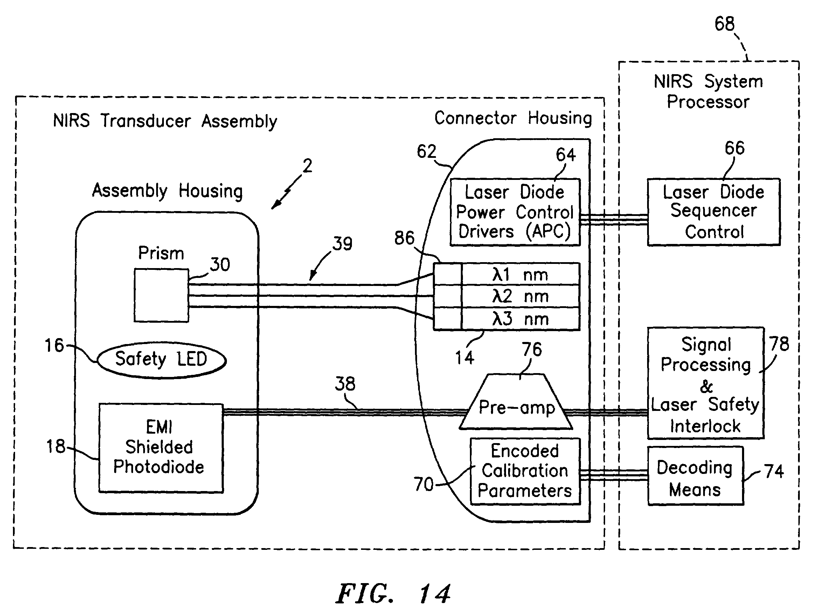

In another alternative connector housing embodiment shown in FIG. 14, fiber optic NIRS probes 39 are employed and the laser diodes 14 are disposed in the connector housing 62. A coupler 86 interconnects the laser diodes 14 with the fiber optic light pipes 39. The automatic power control drivers 64 are also disposed in the connector housing 62, as previously described in the embodiments shown in FIGS. 12 and 13.

The NIRS transducer assembly 2 of this invention operates as follows. The assembly 2 has a multiplexed laser diode firing system in which only one laser diode at a time (emitting one wavelength) is pulsed "ON" and is modulated at a predetermined carrier frequency. The automatic power control (APC) drivers 64 for the laser diodes 14 will also operate at the predetermined modulation rate while maintaining the tight power tolerance. There is a dark period in which all of the laser diodes 14 will be "OFF", allowing for offset voltages to be sampled and subtracted. The duration of the dark period is usually much longer than the time period when the laser diodes are "ON". Thus, the overall duty cycle of the laser diodes is small. The photodiode located in the NIRS probe detects the laser light that irradiated the biological tissue. A transimpedance photodiode preamplifier converts the detected light to a voltage. A band pass filter of predetermined bandwidth, centered on the carrier frequency will be the first step in filtering out noise from the detected signal. Demodulation of the detected signal further attenuates noise and removes the carrier frequency. An adjustable gain amplifier will be used to increase the detected light levels to the desired range. An analog to digital converter demultiplexes each wavelength, the converter being controlled by timing circuitry that is synchronized with the laser diode pulsing. The data obtained is processed by a computer by use of a multi variate Modified Beer-Lambert Law algorithm which calculates the physiological parameters of interest. The determined physiological parameters: $\Delta Hb$, $\Delta HbO_2$, and $\Delta Total\ Hb$ are displayed on a monitor.

The NIRS algorithm employed by the assembly of this invention is based on a multi variate form of the Beer-Lambert Law which is expressed in a matrix form since three laser diodes are employed. Relative changes of the concentrations of $HbO_2$ and $Hb$ can be quantified by using the modified Beer-Lambert Law, which takes into account the optical attenuation in a highly scattering medium like biological tissue. Absolute measurement of chromophore concentration is very challenging because the determination of optical attenuation due to scattering losses is difficult. However, by measuring differential optical attenuation from an initial baseline, optical attenuation due to scattering is canceled out. The multi variate form of the modified Beer-Lambert Law requires that optical attenuation needs to be measured at least two different wavelengths to determine the two unknown chromophores $\Delta HbO_2$ and $\Delta Hb$. If the number of wavelengths used is equal to the number of chromophores of interest, then the solution can be solved by Cramer's Rule. If the number of wavelengths used is greater than the number of chromophores of interest, as in the case of the NIRS system described above, a least squares multi linear regression method is used in the algorithm to solve for the chromophores of interest. In theory, the greater the number of measurement wavelengths the more increased reduction of errors in the determination of the chromophore concentration is achieved. Thus, the use of three laser diodes to measure two chromophores, as described above, will result in a more accurate measurement of the two chromophores. It will appreciated that the system described above could also be modified to measure a third chromophore in biological tissue, such as cytochrome. When fiber optic elements are included in the assembly, the preferred laser diodes are continuous wave vertical cavity surface emitting lasers (VCSELs); and when the fiber optic elements are not included in the assembly, continuous wave edge emitting semiconductor lasers may be used.

Since many changes and variations of the disdosed embodiment of the invention may be made without departing from the inventive concept, it is not intended to limit the invention otherwise than as required by the appended claims.

**Claims**

1. A near infrared spectrophotometric (NIRS) monitoring assembly for non-invasive monitoring of blood oxygenation levels in a subject's body, said assembly comprising:

   a) at least one flexible housing which can be attached directly to a subject's body;
   b) a plurality of laser diodes disposed in said housing, each of said laser diodes being operable to emit a near infrared light signal of a different wavelength; and
   c) a photodiode assembly which can be attached directly to the subject's body and which is operably connected to said laser diodes, said photodiode assembly being operable to measure light intensity values emanating from light emitted by said laser diodes and passing through the subject's body.

2. The NIRS monitoring assembly of Claim 1 wherein said photodiode assembly is disposed in said housing.

3. The NIRS monitoring assembly of Claim 1 or 2 further comprising an auxiliary light emitter disposed in said housing and operable to emit light which can be continuously detected by said photodiode assembly, whereby an increase or a decrease in the intensity of detected light emanating from said auxiliary light emitter outside of a predetermined intensity range, will indicate to said photodiode assembly and to a NIRS system processor that the NIRS monitoring assembly has become detached from the subject's skin.

4. The NIRS monitoring assembly of any of Claims 1 to 3, wherein said photodiode assembly is shielded from ambient electromagnetic interference.

5. The NIRS monitoring assembly of Claim 1 wherein said photodiode assembly is contained in a second housing.

6. The NIRS monitoring assembly of any of Claims 1 to 5, wherein said laser diodes are overlain by a rigid transparent light guide which is positioned in said housing so as to directly contact the subject's skin when said NIRS monitoring assembly is secured to the subject.

7. The NIRS monitoring assembly of Claim 6 wherein said light guide has a planar surface which contacts the subject's skin when the NIRS monitoring assembly is secured to the subject.

8. The NIRS monitoring assembly of any of Claims 1 to 7 further including a disposable sterile adhesive envelope or pad which is interposed between the housing and the subject's skin and which provides adhesion for the NIRS monitoring assembly to the subject's skin.

9. A near infrared spectrophotometric (NIRS) monitoring assembly for non-invasive monitoring of blood oxygenation levels in a subject's body, said assembly comprising:

   a) at least one flexible housing which can be attached directly to a subject's body;
   b) a plurality of laser diodes operably connected to said housing, each of said laser diodes being operable to emit a near infrared light signal of a different wavelength;
   c) a photodiode assembly which can be attached directly to the subject's body and which is operably connected to said laser diodes, said photodiode assembly being operable to measure light intensity values emanating from light emitted by said laser diodes and passing through the subject's body; and
   d) an auxiliary light emitter disposed in said housing and operable to emit light which can be continuously detected by said photodiode assembly, whereby an increase or a decrease in the intensity of detected light emanating from said auxiliary light emitter outside of a predetermined intensity range, will indicate to said photodiode assembly and to a NIRS system processor that the NIRS monitoring assembly has become detached from the subject's skin.

10. The NIRS monitoring assembly of Claim 9 wherein said auxiliary light emitter is a light emitting diode.

11. The NIRS monitoring assembly of Claim 9 or 10, wherein said laser diodes are contained in said housing.

12. The NIRS monitoring assembly of Claim 11 wherein said laser diodes are overlain by a rigid transparent light guide which is positioned in said housing so as to directly contact the subject's skin when said NIRS monitoring assembly is secured to the subject.

**13.** The NIRS monitoring assembly of Claim 12 wherein said light guide has a planar surface which contacts the subject's skin when the NIRS monitoring assembly is secured to the subject.

**14.** A near infrared spectrophotometric (NIRS) monitoring assembly for non-invasive monitoring of blood oxygenation levels in a subject's body, said assembly comprising:

  a) at least one flexible housing for attachment to a subject's body;
  b) a plurality of light sources operably connected to said housing, each of said light sources being operable to emit a near infrared light signal of a different wavelength;
  c) a photodiode assembly for attachment to the subject's body and which is operably connected to said light sources so as to be operable to measure light intensity values emanating from light emitted by said light sources and passing through the subject's body; and
  d) a disposable sterile adhesive envelope or pad which is interposed between the housing and the subject's skin and which provides adhesion for the NIRS monitoring assembly to the subject's skin.

**15.** A near infrared spectrophotometric (NIRS) monitoring assembly for non-invasive monitoring of blood oxygenation levels in a subject's body, said assembly comprising:

  a) at least one flexible housing which can be attached directly to a subject's body;
  b) a plurality of light sources disposed in said housing, each of said laser diodes being operable to emit a near infrared light signal of a different wavelength;
  c) a photodiode assembly which can be attached directly to the subject's body and which is operably connected to said laser diodes, said photodiode assembly being operable to measure light intensity values emanating from light emitted by said light sources and passing through the subject's body; and
  d) an ambient electromagnetic interference (EMI) shield surrounding said photodiode assembly, said EMI shield including a conductive mesh screen interposed between said photodiode assembly and a surface of the NIRS monitoring assembly that abuts the subject's skin when the assembly is attached to the subject's body, and an electrically conductive capsule which covers all surfaces of said photodiode assembly which are not covered by said mesh screen, said mesh screen and said capsule allowing measurement of light intensity values emitted by said light sources while preventing ambient EMI from influencing operation of said NIRS monitoring assembly.

**16.** A near infrared spectrophotometric (NIRS) monitoring assembly for non-invasive monitoring of blood oxygenation levels in a subject's body, said assembly comprising:

  a) at least one flexible housing which can be attached directly to a subject's body;
  b) a plurality of laser diodes disposed in said housing, each of said laser diodes being operable to emit a near infrared light signal of a different wavelength, said laser diodes being overlain by a rigid transparent light guide which is positioned in said housing so as to directly contact the subject's skin when said NIRS monitoring assembly is secured to the subject.; and
  c) a photodiode assembly which can be attached directly to the subject's body and which is operably connected to said laser diodes, said photodiode assembly being operable to measure light intensity values emanating from light emitted by said laser diodes and passing through the subject's body.

**17.** A near infrared spectrophotometric (NIRS) monitoring assembly for non-invasive monitoring of blood oxygenation levels in a subject's body, said assembly comprising:

  a) at least one flexible housing which can be attached directly to a subject's body, said housing including a transparent window;
  b) a plurality of light sources which are operably associated with said housing, each of said light sources being operable to emit a near infrared light signal of a different wavelength, which light signals are transmitted through said housing window;
  c) a photodiode assembly which can be attached directly to the subject's body and which is operable to measure light intensity signals emanating from light emitted by said light sources after passing through the subject's body;
  d) a processor which controls actuation of said light sources and which processes light intensity signals from said photodiode assembly; and
  e) a connector housing assembly interposed between said processor and said light sources and photodiode assembly, said connector housing assembly including light source power driver controls connected to said processor and connected to said light sources, and an encoding calibration mechanism connected to said processor for supplying essential NIRS operating calibrating information to said processor.

**18.** The NIRS assembly of Claim 17 further including a photodiode preamplifier interposed between said processor and said photodiode.

**19.** The NIRS assembly of Claim 17 or 18, wherein said light source power driver controls are adjustable.

**20.** A near infrared spectrophotometric (NIRS) monitoring assembly for non-invasive monitoring of blood oxygenation levels in a subject's body, said assembly comprising:

a) at least one flexible housing which can be attached directly to a subject's body;
b) a plurality of laser diodes disposed in said housing, each of said laser diodes being operable to emit a near infrared light signal of a different wavelength;
c) a photodiode assembly which can be attached directly to the subject's body and which is operably connected to said laser diodes, said photodiode assembly being operable to measure light intensity values emanating from light emitted by said laser diodes and passing through the subject's body;
d) a processor which controls actuation of said laser diodes and which processes light intensity signals from said photodiode assembly; and
e) a thermistor positioned adjacent to said laser diodes, said thermistor being operably connected to said processor, and said thermistor being operable to monitor laser diode temperature and provide laser diode operating temperature information to said processor for compensating for wavelength and power shifting of said laser diodes.

**FIG. 1**

**FIG. 2**

**FIG. 3**

FIG. 4

FIG. 4A

FIG. 5

FIG. 6

FIG. 7

FIG. 8

FIG. 9

FIG. 10

FIG. 11

FIG. 11A

*FIG. 12*

**FIG. 13**

EP 1 080 683 A2

**FIG. 14**